Europäisches Patentamt

European Patent Office

Office européen des brevets

(11) Veröffentlichungsnummer: **0 225 843**
**B1**

(12) **EUROPÄISCHE PATENTSCHRIFT**

(45) Veröffentlichungstag der Patentschrift:
26.09.90

(51) Int. Cl.⁵: **A61K 6/10**

(21) Anmeldenummer: 86810509.9

(22) Anmeldetag: 07.11.86

(54) **Härtbares Silikonabformmaterial, welches beim Mischen eine optische Kontrolle des ausreichenden Durchmischens erlaubt.**

(30) Priorität: 22.11.85 CH 5003/85

(43) Veröffentlichungstag der Anmeldung:
16.06.87 Patentblatt 87/25

(45) Bekanntmachung des Hinweises auf die Patenterteilung:
26.09.90 Patentblatt 90/39

(84) Benannte Vertragsstaaten:
DE FR GB IT NL SE

(56) Entgegenhaltungen:
EP-A- 0 158 141
GB-A- 776 501
US-A- 2 816 843
US-A- 3 081 177

Römpps Chemie-Lexikon, 8. Auflage,
Seiten 522, 1864, 4661

(73) Patentinhaber: Coltène AG, Feldwiesenstrasse 20,
CH-9450 Altstätten (SG)(CH)

(72) Erfinder: Nalbantova, Stefka, Widenelsch 10,
CH-9450 Altstätten(CH)
Erfinder: Kägi, Stephan, Dr., Naglerstrasse 11a,
CH-9443 Widnau(CH)

(74) Vertreter: Frauenknecht, Alois J. et al, c/o PPS
Polyvalent Patent Service AG, Mellingerstrasse 1,
CH-5400 Baden(CH)

## Beschreibung

Die vorliegende Erfindung bezieht sich auf eine härtbare Masse, basierend auf einem Silikonabformmaterial, welche Masse beim Mischen eine optische Kontrolle des ausreichenden Durchmischens erlaubt, wobei deren Komponenten unter vorgegebenen Bedingungen solange gemischt werden, bis über die gesamte beobachtbare Oberfläche der Masse, in bezug auf die Farbe wenigstens einer ursprünglichen Komponente, ein Farbumschlag erreicht wird.

Die härtbare Masse wird insbesondere im zahnärztlichen oder technischen Bereich zur Abformung mit Elastomeren aber auch im Klebe- und Dichtungsbereich verwendet.

Es ist allgemein bekannt, daß aus wenigstens zwei Komponenten bestehende härtbare Massen sehr gut gemischt werden müssen. Aus dem Buch Rehberg "Die Quintessenz der zahnärztlichen Abformhilfsmittel", 1. Auflage, Quintessenz Verlag Berlin 1971, Seiten 90 und 91, ist ersichtbar, daß die meisten Fehler bei der Vorbereitung von als Zahnabformmaterialien dienenden härtbaren Massen durch ungenügende Mischung entstehen und somit fehlerhafte Abdrücke verursachen. Dieses Problem ist schon lange bekannt. Um in einzelnen Schichten von Abformmaterialien oder anderen härtbaren Massen die sogenannten "Zwiebelringe und Blätterteige" zu verhindern, wurde z.B. folgende Methode vorgeschlagen: Das Basismaterial ist in einer Farbe gefärbt, der Flüßig- oder Pastenhärter in einer anderen. So ist z.B. das Basismaterial gelb und der Pastenhärter blau. Beim gemeinsamen Mischen entsteht infolge des subtraktiven Mischens der Farben die grüne Farbe. Bei dieser Form der visuellen Mischkontrolle handelt es sich um ein reines Verteilen des Farbstoffes in der Masse. Die Mischung wird aufgrund der Farbverteilung beurteilt. Diese Methode hat jedoch den Nachteil, daß bei einem nicht ausreichenden Durchmischen die verschiedenen Intensitäten der grünen Farbe nur schlecht zu unterscheiden sind und daß somit auch eine fehlerhaft durchgemischte Maße als eine richtig bearbeitete Masse betrachtet werden kann.

Es ist ferner bekannt (US-A 2 816 843, GB-A 776 501 und US-A 3 081 177), bei der Herstellung von Dental Abformmassen aus Alginatmischungen einen Base-Säure-Indikator beizugeben, um die rapide Gelierungsreaktion zu verflogen. Die auf Alginaten basierenden Abformmassen benötigen Anmachwasser für deren Gelierung, so daß die zugegebenen pH-Indikatoren den Gelierzustand und damit das ausreichende Durchmischen anzuzeigen vermögen. Das vorgängig benötigte Wasser wird in die abgebundene Masse miteingebaut, so daß Dentalabdrücke resultieren mit einem relativ schlechten Langzeitverhalten, weswegen diese sofort ausgegossen werden müssen. Bessere physikalische Eigenschaften zeigen dagegen silikonelastomere Abformmassen, welche bekanntlich nicht-wäßrige Systeme darstellen und daher besser für Anwendungen, insbesondere im Dentalbereich, geeignet sind.

Der folgenden Erfindung liegt die Aufgabe zugrunde, eine härtbare Masse, basierend auf Silikonabformmaterialien, zu schaffen, bei der die richtige Durchmischung auch durch eine weniger geübte Person sicher und fehlerfrei festgestellt werden kann.

Die obengenannte Aufgabe wird dadurch gelöst, daß wenigstens eine der Komponenten eine homogen zugemischte Substanz enthält, die beim vollständigen Durchmischen mit der weiteren Komponente einen Farbumschlag hoher Intensität und/oder eines hohen Kontrasts ergibt.

Das Verwenden wenigstens einer Substanz, mit der man einen Farbumschlag erreichen kann, ermöglichte die Endphase des Mischens präzise zu erkennen. Beispielsweise wird bei einer farblosen Basis und einem Härter, der die farbumschlagende Substanz enthält, die Endphase des Mischvorganges an der Tatsache erkannt, daß die ganze härtbare Masse z.B. blau wird. Damit kann visuell gezeigt werden, daß die Komponenten in Wechselwirkung getreten sind und eine Reaktion stattfand. Der Farbkontrast erlaubt eine wesentlich sichere Beurteilung der Mischergebnisse, da die aktuelle Farbe aktiv durch einen Farbumschlag gebildet wird. Es entstehen also keine Übergangszonen der Farbenintensität, wie es bei einem nur subtraktiven Mischen der Farben der Fall wäre.

Ein weiterer Vorteil ist darin zu sehen, daß während des Mischens der Komponenten eine Farbreaktion abläuft, welche schneller ist als der Mischvorgang selbst. Auf diese Weise kann gewährleistet werden, daß der momentane Mischzustand des Abformmaterials immer präzise angezeigt wird. Die Raktanden der Farbreaktion können entweder speziell zugegeben werden oder aber auch Bestandteil der Komponenten sein.

Konkrete Weiterentwicklungen der Erfindungsidee sind in den Unteransprüchen beschrieben.

Gemäß Anspruch 2 verwendet man als die farbumschlagende Substanz einen oder mehrere pH-Indikatoren. Der Umschlag erfolgt durch eine Änderung ihrer sauren oder basischen Bestandteile. Als Beispiel wird Bromphenolblau erwähnt.

Gemäß einer anderen Variante nach Anspruch 3 wird als die wenigstens eine farbumschlagende Substanz ein Metall-Indikator, z.B. in den Härter, eingemischt. Für diese Variante kann man als Beispiel folgende Substanzen nennen: Erio-Chromschwarz-T, Dithizon und weitere bekannte Indikatoren, wie sie z.B. in J. Fries, H. Getrost, "Organische Reagenzien für die Spurenanalyse", Merck Darmstadt, 1977, erwähnt sind.

Eine günstige Möglichkeit nach Anspruch 4 besteht darin, daß man als farbumschlagende Substanz einen Redox-Indikator verwendet, z.B. Methylenblau aber auch Küpenfarbstoffe resp. ihre Leucoverbindung oder Eisen II Rhodanid.

Der Farbumschlag erfolgt durch Oxidation oder Reduktion der farbumschlagenden Substanz.

Es ist zweckmäßig, wenn nach Anspruch 5 und 6 die wenigstens eine farbumschlagende Substanz ein Fluoreszenz- oder Adsorptionsindikator ist (z.B. Eosin). Diese Indikatoren sind meistens zum mindesten in einer Komponente, z.B. im Härter, leicht löslich und der Farbumschlag findet sehr schnell statt.

Gemäß einer vorteilhaften Weiterausbildung des Erfindungsgegenstandes nach Anspruch 7 enthält eine Komponente Jod und eine andere Komponente Stärke.

Nach Anspruch 8 wird als Farbumschlagreaktion eine Komplexbildungsreaktion gewählt. Als Ausgangsmaterialien können einfach Metallionen und Komplexbildner eingesetzt werden, z.B. Ni(II) und Dimethylglyoxim. Es ist aber auch möglich, bereits von Metallkomplexen auszugehen und weitere Metallionen zu inkorporieren, z.B. Berliner Blau oder Turnbulls Blau oder an zwei oder mehreren Komplexen die Liganden zu tauschen, z.B. Mg(EDTA) und Fe(III) Rhodanid.

Die Farbstoffsynthese nach Anspruch 9 erfolgt nach dem gleichen Prinzip wie z.B. die Farbkuppler in der Farbphotographie. Bei den dort verwendeten Substanzen handelt es sich hauptsächlich um Polymethinfarbstoffe; es sind aber auch andere Additionsprinzipien denkbar.

Aus den folgenden Beispielen sind Zusammensetzungen und Herstellungsverfahren, sowohl von einzelnen Komponenten als auch von fertigen härtbaren Massen, insbesondere von Zahnabdruckmaterialien, zu entnehmen.

Beispiele

A. Zusammensetzung und Herstellung von Basispasten

A1. Es wird eine Basispaste eines Silikonabformmaterials hergestellt, in dem
80 Teile Dimethylpolysiloxan
15 Teile Calciumcarbonat
2 Teile hochdisperse Kieselsäure
in einem Mischer während zwei Stunden miteinander vermischt werden. Diese weisse Basispaste dient auch als Grundlage für nachfolgend beschriebene Basispasten.

A2. 100 Teile der Basispaste von Beispiel A1 werden mit 0,5 Teilen einer gesättigten Stärkelösung homogen vermischt. Es entsteht eine weisse Masse, die eine andere Basismasse bildet.

A3. 50 Teile der Basispaste von Beispiel A1 werden mit 0,05 Teilen einer warmen konzentrierten Nickelnitratlösung versetzt und bis zur Homogenität vermischt. Es entsteht eine weisse Basispaste.

B. Zusammensetzung und Herstellung von Härtern

B1. Es wird ein Härter eines Silikonabformmaterials aus
20 Teilen oligomerem Kieselsäureester
10 Teilen Dibutylzinndilaurat
20 Teilen gesättigten Kohlenwasserstoffen
homogen zusammengemischt. Dieser farblose Härter wird als Grundlage für nachfolgend beschriebene Härter verwendet.

B2. Zu 50 Teilen des Härters von Beispiel B1 werden 0,1 Teile Bromphenolblau gegeben und homogen gelöst. Es resultiert ein roter Härter.

B3. In 100 Teilen des Härters von Beispiel B1 werden 0,5 Teile Jod homogen gelöst. Es resultiert ein gelbbrauner Härter.

B4. 40 Teile des Härters von Beispiel B1 werden unter Rühren mit 0,1 Teil Eosin homogen gelöst. Es entsteht ein rot-oranger Härter.

B5. 20 Teile des Härters von Beispiel B1 werden mit 0,1 Teil Dimethylglyoxim bis zur Homogenität gerührt. Es entsteht ein farbloser Härter.

C. Zusammensetzung und Herstellung von härtbaren Massen insbesondere von Zahnabformmaterialien

C1. Umschlag mittels Jod-Stärke: 10 Teile der weissen Basispaste von Beispiel A2 werden mit 1 Teil des gelb-braunen Härters von Beispiel B3 zusammengemischt. Beim Mischen findet ein Farbumschlag zu blau statt.

C2. Umschlag durch eine Komplexbildung: 10 Teile der weissen Basispaste von Beispiel A3 werden mit 1 Teil des Härters von Beispiel B5 angemischt. Während des Mischens verfärbt sich die härtbare Masse rosa.

C3. Umschlag aufgrund eines pH-Indikators: 10 Teile der Basispaste von Beispiel A1 werden mit 1 Teil des roten Härters von Beispiel B2 angerührt. Die Farbe der härtbaren Masse wechselt zu blau.

C4. Umschlag durch Adsoprtion: 10 Teile der Basispaste von Beispiel A1 werden mit 1 Teil des Härters von Beispiel B4 zusammengemischt. Beim Zahnabdruckmaterial findet ein Farbumschlag nach blau-rosa statt.

Die erfindungsgemässen Silikonabformmaterialien weisen eine gesteigerte Qualität, insbesondere des Zahnabdruckmaterials, auf, ohne zusätzlichen Arbeitsaufwand für die Person, die die härtbare Masse bearbeitet.

Patentansprüche

1. Härtbares Silikonabformmaterial, insbesondere für zahnärztliche Anwendungen, bestehend aus wenigstens zwei Komponenten, welches beim Mischen eine optische Kontrolle des ausreichenden Durchmischens erlaubt, wobei den Komponenten unter vorgegebenen Bedingungen so lange gemischt werden, bis über die gesamte beobachtbare Oberfläche des Materials, in bezug auf die Farbe wenigstens einer ursprünglichen Komponente, ein Farbumschlag erreicht wird, dadurch gekennzeichnet, daß wenigstens eine dieser Komponenten eine homogen zugemischte Substanz enthält, die beim vollständigen Durchmischen mit der weiteren Komponente einen Farbumschlag hoher Intensität und/oder eines hohen Kontrasts ergibt.

2. Härtbares Silikonabformmaterial nach Anspruch 1, dadurch gekennzeichnet, daß wenigstens eine farbumschlagende Substanz aus der Gruppe der pH-Indikatoren stammt.

3. Härtbares Silikonabformmaterial nach Anspruch 1, dadurch gekennzeichnet, daß wenigstens eine farbumschlagende Substanz aus der Gruppe der Metall-Indikatoren stammt.

4. Härtbares Silikonabformmaterial nach Anspruch 1, dadurch gekennzeichnet, daß wenigstens eine farbumschlagende Substanz aus der Gruppe der Redox-Indikatoren stammt.

5. Härtbares Silikonabformmaterial nach Anspruch 1, dadurch gekennzeichnet, daß wenigstens eine farbumschlagende Substanz aus der Gruppe der Adsorptions-Indikatoren stammt.

6. Härtbares Silikonabformmaterial nach Anspruch 1, dadurch gekennzeichnet, daß wenigstens eine farbumschlagende Substanz aus der Gruppe der Fluoreszenzindikatoren stammt.

7. Härtbares Silikonabformmaterial nach Anspruch 1, dadurch gekennzeichnet, daß wenigstens eine der Komponenten Jod und eine andere Komponente Stärke enthalten.

8. Härtbares Silikonabformmaterial nach Anspruch 1, dadurch gekennzeichnet, daß wenigstens eine Komponente einen Komplexbildner und eine andere reaktionsfähige Metallionen enthält.

9. Härtbares Silikonabformmaterial nach Anspruch 1, dadurch gekennzeichnet, daß zwei Komponenten wenigstens je eine Substanz enthalten, welche sich beim Vermischen zu einem Farbstoff verbinden.

**Claims**

1. Hardenable silicone casting material, in particular for dental applications, comprising at least two components which, upon mixing, permits a visual check of sufficient blending, its components being mixed under predetermined conditions until a colour change is achieved over the entire observable surface of the material with respect to the colour of at least one original component, characterized in that at least one of these components contains a homogeneously admixed substance which, upon complete blending with the other component, results in a colour change of high intensity, and/or high contrast.

2. Hardenable silicone casting material according to Claim 1, characterized in that at least one substance undergoing colour change is from the group comprising pH indicators.

3. Hardenable silicone casting material according to Claim 1, characterized in that at least one substance undergoing colour change is from the group comprising metal indicators.

4. Hardenable silicone casting material according to Claim 1, characterized in that at least one substance undergoing colour change is from the group comprising redox indicators.

5. Hardenable silicone casting material according to claim 1, characterized in that at least one substance undergoing colour change is from the group comprising adsorption indicators.

6. Hardenable silicone casting material according to Claim 1, characterized in that at least one substance undergoing colour change is from the group comprising fluorescence indicators.

7. Hardenable silicone casting material according to claim 1, characterized in that at least one of the components contains iodine and another component contains starch.

8. Hardenable silicone casting material according to claim 1, characterized in that at least one component contains a complex former and another contains reactive metal ions.

9. Hardenable silicone casting material according to Claim 1, characterized in that two components each contain at least one substance which on mixing combine to form a dye.

**Revendications**

1. Matériau de moulage durcissable à base de silicone, en particulier pour des applications dentaires, composé d'au moins deux composants et permettant lors du mélange un contrôle optique du processus de mélange, ses composants étant mélangés dans des conditions prédéterminées jusqu'à ce qu'un changement de couleur soit atteint sur toute la surface observable du matériau par rapport à la couleur d'au moins l'un des composants d'origine, caractérisé en ce qu'au moins l'un des composants contient une substance ajoutée de manière homogène qui, au cours du mélange complet avec l'autre composant, provoque un changement de couleur d'une forte intensité et/ou d'un fort contraste.

2. Matériau de moulage durcissable à base de silicone selon la revendication 1, caractérisé en ce qu'au moins une substance provoquant le changement de couleur appartient au groupe des indicateurs de pH.

3. Matériau de moulage durcissable à base de silicone selon la revendication 1, caractérisé en ce qu'au moins une substance provoquant le changement de couleur appartient au groupe des indicateurs de métalliques.

4. Matériau de moulage durcissable à base de silicone selon la revendication 1, caractérisé en ce qu'au moins une substance provoquant le changement de couleur appartient au groupe des indicateurs de redox.

5. Matériau de moulage durcissable à base de silicone selon la revendication 1, caractérisé en ce qu'au moins une substance provoquant le changement de couleur appartient au groupe des indicateurs d'adsorption.

6. Matériau de moulage durcissable à base de silicone selon la revendication 1, caractérisé en ce qu'au moins une substance provoquant le changement de couleur appartient au groupe des indicateurs de fluorescence.

7. Matériau de moulage durcissable à base de silicone selon la revendication 1, caractérisé en ce qu'au moins l'une des composants contient de l'iode et que l'autre composant contient de l'amidon.

8. Matériau de moulage durcissable à base de silicone selon la revendication 1, caractérisé en ce qu'au moins l'une des composants contient un agent complexant et que l'autre composant contient des ions métalliques qui entrent facilement en réaction.

9. Matériau de moulage durcissable à base de silicone selon la revendication 1, caractérisé en ce que deux composants contiennent chacun au moins une substance lesquelles se combinent lors du mélange en un colorant.